# EUROPEAN PATENT APPLICATION

(11) **EP 1 422 239 A2**
(43) Date of publication of application: **26.05.2004**
(21) Application number: 03022784.7
(22) Date of filing: 23.10.1996
(51) Int. Cl.: C07K 14/52, A61K 38/19

(54) **Mobilization of hematopoietic stem cells using a chemokine**

(30) Priority: 24.10.1995 US 6051 P
(62) Divisional of application: 96936873.7
(71) Applicant: SMITHKLINE BEECHAM CORPORATION, King of Prussia, PA 19406-0939 (US); Human Genome Sciences, Inc., Rockville, MD 20850 (US)
(72) Inventor: White, John, Coatesville pa 19320 (US); Pelus, Louis, Richboro PA 18954 (US); Li, Haodong, Gaithersburg MD 20878 (US); Kreider, Brent L., Germantown MD 20874 (US)
(74) Representative: Crump, Julian Richard John

(57) **Abstract**

Novel chemokines for mobilizing stem cells are provided. Methods of mobilizing stem cells are also provided.

SEQUENCE LISTING

<110> SmithKline Beecham Corporation
      Human Genome Sciences, Inc

<120> Mobilization of Hematopoietic Stems Cells Using a Chemokine

<130> 40244

<150> US 60/006051

<151> 1995-10-24

<160> 18

<210> 1

<211> 70

<212> PRT

<213> CKBeta-4

<400> 1

Ala Ser Asn Phe Asp Cys Cys Leu Gly Tyr Thr Asp Arg Ile Leu
1               5                   10                  15

His Pro Lys Phe Ile Val Gly Phe Thr Arg Gln Lue Ala Asn Glu
                20                  25                  30

Gly Cys Asp Ile Asn Ala Ile Ile Phe His Thr Lys Lys Lys Leu
                35                  40                  45

Ser Val Cys Ala Asn Pro Lys Gln Thr Trp Val Lys Tyr Ile Val
                50                  55                  60

Arg Leu Leu Ser Lys Lys Val Lys Asn Met
                65                  70

<210> 2

<211> 70

<212> PRT

<213> CKBeta-6

<400> 2

Val Val Ile Pro Ser Pro Cys Cys Met Phe Phe Val Ser Lys Arg
1               5                   10                  15

Ile Pro Glu Asn Arg Val Val Ser Tyr Gln Lue Ser Ser Arg Ser
                20                  25                  30

Thr Cys Leu Lys Gly Gly Val Ile Phe Thr Thr Lys Lys Gly Gln
                35                  40                  45

Gln Phe Cys Gly Asp Pro Lys Gln Glu Trp Val Gln Arg Tyr Met
                50                  55                  60

Lys Asn Leu Asp Ala Lys Gln Lys Lys Ala
                65                  70

<210> 3

<211> 60

<212> PRT

<213> CKBeta-7

<400> 3

Ala Gln Val Gly Thr Asn Lys Glu Leu Cys Cys Leu Val Tyr Thr
1               5                   10                  15

Ser Trp Gln Ile Pro Gln Lys Phe Ile Val Asp Tyr Ser Glu Thr
                20                  25                  30

Ser Pro Gln Cys Pro Lys Pro Gly Val Ile Leu Leu Thr Lys Arg
                35                  40                  45

Gly Arg Gln Ile Cys Ala Asp Pro Asn Lys Lys Trp Val Gln Lys
                50                  55                  60

<210> 4

<211> 82

<212> PRT

<213> CKBeta-8

<400> 4

Glu Asn Pro Val Leu Leu Asp Arg Phe His Ala Thr Ser Ala Asp
1               5                   10                  15

Cys Cys Ile Ser Tyr Thr Pro Arg Ser Ile Pro Cys Ser Leu Leu
                20                  25                  30

Glu Ser Tyr Phe Glu Thr Asn Ser Glu Cys Ser Lys Pro Gly Val
                35                  40                  45

Ile Phe Leu Thr Lys Lys Gly Arg Arg Phe Cys Ala Asn Pro Ser
                50                  55                  60

Asp Lys Gln Val Gln Val Cys Met Arg Met Leu Lys Leu Asp Thr
                65                  70                  75

Arg Ile Lys Thr Arg Lys Asn
                80

<210> 5

<211> 79

<212> PRT

<213> CKBeta-9


<400> 5

Ser Asp Ala Gly Gly Ala Gln Asp Cys Cys Leu Lys Tyr Ser Gln
1               5                   10                  15

Arg Lys Ile Pro Ala Lys Val Val Arg Ser Tyr Arg Lys Gln Glu
                20                  25                  30

Pro Ser Leu Gly Cys Ser Ile Pro Ala Ile Leu Phe Leu Pro Arg
                35                  40                  45

Lys Arg Ser Gln Ala Glu Leu Cys Ala Asp Pro Lys Glu Leu Trp
                50                  55                  60

Val Gln Gln Leu Met Gln His Leu Asp Lys Thr Pro Ser Pro Gln
                65                  70                  75

Lys Pro Ala Gln


<210> 6

<211> 82

<212> PRT

<213> CKBeta-10


<400> 6

Phe Asn Pro Gln Gly Leu Ala Gln Pro Asp Ala Leu Asn Val Pro
1               5                   10                  15

Ser Thr Cys Cys Phe Thr Phe Ser Ser Lys Lys Ile Ser Leu Gln
                20                  25                  30

Arg Leu Lys Ser Tyr Val Ile Thr Thr Ser Arg Cys Pro Gln Lys
                35                  40                  45

Ala Val Ile Phe Arg Thr Lys Leu Gly Lys Glu Ile Cys Ala Asp
                50                  55                  60

Pro Lys Glu Lys Trp Val Gln Asn Tyr Met Lys His Leu Gly Arg
                65                  70                  75

Lys Ala His Thr Leu Lys Thr
                80


<210> 7

<211> 83

<212> PRT

<213> CKBeta-11

<400> 7

Pro Ala Pro Thr Leu Ser Gly Thr Asn Asp Ala Glu Asp Cys Cys
1               5                   10                  15

Leu Ser Val Thr Gln Lys Pro Ile Pro Gly Tyr Ile Val Arg Asn
                20                  25                  30

Phe His Tyr Leu Leu Ile Lys Asp Gly Cys Arg Val Pro Ala Val
                35                  40                  45

Val Phe Thr Thr Leu Arg Gly Arg Gln Leu Cys Ala Pro Pro Asp
                50                  55                  60

Gln Pro Trp Val Glu Arg Ile Ile Gln Arg Leu Gln Arg Thr Ser
                65                  70                  75

Ala Lys Met Lys Arg Arg Ser Ser
                80

<210> 8

<211> 82

<212> PRT

<213> CKBeta-12

<400> 8

Arg Ser Gln Pro Lys Val Pro Glu Trp Val Asn Thr Pro Ser Thr
1               5                   10                  15

Cys Cys Leu Lys Tyr Tyr Glu Lys Val Leu Pro Arg Arg Leu Val
                20                  25                  30

Val Gly Tyr Arg Lys Ala Leu Asn Cys His Leu Pro Ala Ile Ile
                35                  40                  45

Phe Val Thr Lys Arg Asn Arg Glu Val Cys Thr Asn Pro Asn Asp
                50                  55                  60

Asp Trp Val Gln Glu Tyr Ile Lys Asp Pro Asn Leu Pro Leu Leu
                65                  70                  75

Pro Thr Arg Asn Leu Ser Thr
                80

<210> 9

<211> 68

<212> PRT

<213> CKBeta-13

<400> 9

Pro Tyr Gly Ala Asn Met Glu Asp Ser Val Cys Cys Arg Asp Tyr
1               5                   10                  15

Val Arg Tyr Arg Leu Pro Leu Arg Val Val Lys His Phe Tyr Trp
                20                  25                  30

Thr Ser Asp Ser Cys Pro Arg Pro Gly Val Val Leu Leu Thr Phe
                                    35                  40  45

Arg Asp Lys Glu Ile Cys Ala Asp Pro Arg Val Pro Trp Val Lys
                50                  55                  60

Met Ile Leu Asn Lys Leu Ser Gln
                65

<210> 10

<211> 69

<212> PRT

<213> hRANTES

<400> 10

Ala Ser Pro Tyr Ser Ser Asp Thr Thr Pro Cys Cys Phe Ala Tyr
1               5                   10                  15

Ile Ala Arg Pro Leu Pro Arg Ala His Ile Lys Glu Tyr Phe Tyr
                20                  25                  30

Thr Ser Gly Lys Cys Ser Asn Pro Ala Val Val Phe Val Thr Arg
                35                  40                  45

Lys Asn Arg Gln Val Cys Ala Asn Pro Glu Lys Lys Trp Val Arg
                50                  55                  60

Glu Tyr Ile Asn Ser Leu Glu Met Ser
                65

<210> 11

<211> 69

<212> PRT

<213> mMIP-1alpha

<400> 11

Ala Pro Tyr Gly Ala Asp Thr Pro Thr Ala Cys Cys Phe Ser Tyr
1               5                   10                  15

Ser Arg Lys Ile Pro Arg Gln Phe Ile Val Asp Tyr Phe Glu Thr
                20                  25                  30

Ser Ser Leu Cys Ser Gln Pro Gly Val Ile Phe Leu Thr Lys Arg
                35                  40                  45

Asn Arg Gln Ile Cys Ala Asp Ser Lys Glu Thr Trp Val Gln Glu
                50                  55                  60

Tyr Ile Thr Asp Leu Glu Leu Asn Ala
                65

<210> 12

<211> 69

<212> PRT

<213> mMIP-1beta

<400> 12

Ala Pro Met Gly Ser Asp Pro Pro Thr Ser Cys Cys Phe Ser Tyr
1               5                   10                  15

Thr Ser Arg Gln Leu His Arg Ser Phe Val Met Asp Tyr Tyr Glu
                20                  25                  30

Thr Ser Ser Leu Cys Ser Lys Pro Ala Val Val Phe Leu Thr Lys
                35                  40                  45

Arg Gly Arg Gln Ile Cys Ala Asn Pro Ser Glu Pro Trp Val Thr
                50                  55                  60

Glu Tyr Met Ser Asp Leu Glu Leu Asn
                65

<210> 13

<211> 78

<212> PRT

<213> MCP-1

<400> 13

Leu Ala Gln Pro Asp Ala Ile Asn Ala Pro Val Thr Cys Cys Tyr
1               5                   10                  15

Asn Phe Thr Asn Arg Lys Ile Ser Val Gln Arg Leu Ala Ser Tyr
                20                  25                  30

Arg Arg Ile Thr Ser Ser Lys Cys Pro Lys Glu Ala Val Ile Phe
                35                  40                  45

Lys Thr Ile Val Ala Lys Glu Ile Cys Ala Asp Pro Lys Gln Lys
                50                  55                  60

Trp Val Gln Asp Ser Met Asp His Leu Asp Lys Gln Thr Gln Thr
                65                  70                  75

Pro Lys Thr

<210> 14

<211> 82

<212> PRT

<213> MCP-3

<400> 14

Ser Pro Gln Gly Leu Ala Gln Pro Val Gly Ile Asn Thr Ser Thr
1               5                   10                  15

Thr Cys Cys Tyr Arg Phe Ile Asn Lys Lys Ile Pro Lys Gln Arg
                20                  25                  30

Leu Glu Ser Tyr Arg Arg Thr Thr Ser Ser His Cys Pro Arg Glu
                35                  40                  45

Ala Val Ile Phe Lys Thr Lys Leu Asp Lys Glu Ile Cys Ala Asp
                50                  55                  60

Pro Thr Gln Lys Trp Val Gln Asp Phe Met Lys His Leu Asp Lys
                65                  70                  75

Lys Thr Gln Thr Pro Lys Leu
                80

<210> 15

<211> 72

<212> PRT

<213> NAP-1/IL-8


<400> 15


Ser Ala Lys Glu Leu Arg Cys Gln Cys Ile Lys Thr Tyr Ser Lys
1               5                   10                  15

Pro Phe His Pro Lys Phe Ile Lys Glu Leu Arg Val Ile Glu Ser
                20                  25                  30

Gly Pro His Cys Ala Asn Thr Glu Ile Ile Val Lys Leu Ser Asp
                35                  40                  45

Gly Arg Glu Leu Cys Leu Asp Pro Lys Glu Asn Trp Val Gln Arg
                50                  55                  60

Val Val Glu Lys Phe Leu Lys Arg Ala Glu Asn Ser
                65                  70

<210> 16

<211> 71

<212> PRT

<213> NAP-2


<400> 16

Ala Glu Leu Arg Cys Met Cys Ile Lys Thr Thr Ser Gly Ile His
1               5                   10                  15

Pro Lys Asn Ile Gln Ser Leu Glu Val Val Ile Gly Lys Gly Thr
            20                  25                  30

His Cys Asn Gln Val Glu Val Ile Ala Thr Leu Lys Asp Gly Arg
                35                  40                  45
Lys Ile Cys Leu Asp Pro Asp Ala Pro Arg Ile Lys Lys Ile Val
                50                  55                  60

Gln Lys Lys Leu Ala Gly Asp Glu Ser Ala Asp
                65                  70

<210> 17

<211> 69

<212> PRT

<213> hPF4

<400> 17

Glu Ala Glu Glu Asp Gly Asp Leu Gln Cys Leu Cys Val Lys Thr
1               5                   10                  15

Thr Ser Gln Val Arg Pro Arg His Ile Thr Ser Leu Glu Val Ile
                20                  25                  30

Lys Ala Gly Pro His Cys Pro Thr Ala Gln Leu Ile Ala Thr Leu
                35                  40                  45

Lys Asn Gly Arg Lys Ile Cys Leu Asp Leu Gln Ala Pro Leu Tyr
                50                  55                  60

Lys Lys Ile Leu Lys Lys Leu Glu Ser
                65

<210> 18

<211> 72

<212> PRT

<213> CKAlpha-1

<400> 18

Val Leu Glu Val Tyr Tyr Thr Ser Leu Arg Cys Arg Cys Val Gln
1               5                   10                  15

Glu Ser Ser Val Phe Ile Pro Arg Arg Phe Ile Asp Arg Ile Gln
                20                  25                  30

Ile Leu Pro Arg Gly Asn Gly Cys Pro Arg Lys Glu Ile Ile Val
                35                  40                  45

Trp Lys Lys Asn Lys Ser Ile Val Cys Val Asp Pro Gln Ala Glu
                50                  55                  60

Trp Ile Gln Arg Met Met Glu Val Leu Arg Lys Arg
                65                  70

## Description

Hematopoietic cells have very important roles in a number of different processes in the body. For example, leukocytic hematopoietic cells are important in maintaining the body's defenses against disease; monocytes, macrophages and lymphocytes are involved in potentiating the body's responses to infection and tumors, while granulocytes are involved in overcoming infection, parasites and tumors. Platelets, another hematopoietic cell, form an important element in the hemostatic mechanism through initiating thrombus formation by their adhesion to each other and to damaged surfaces, and by the release of factors which assist in the formation of the fibrin clot. Erythrocytes are mainly involved in the transport of oxygen.

All of these blood cells are derived from a single progenitor cell called the hematopoietic stem cell. Stem cells are both pluripotent, in that they give rise to all different cell types, and capable of self renewal. Hematopoietic stem cells make up only a small percentage of bone marrow cells and are normally quiescent. However, when stimulated to divide, these stem cells produce a differentiated daughter cell with great proliferative potential. Sequential rounds of division and differentiation give rise to an enormous amplification of cell numbers which is necessary for the production of mature blood cells. This process of division and differentiation is subject to regulation at many levels to control cell production.

Numerous studies have led to the definition of functions of several hematopoietic regulatory messengers. These biomolecules have been characterized as stimulatory, e.g., Colony Stimulating Factors (CSFs) and interleukins (IL-1, IL-3, IL-5 and IL-9); inhibitory, e.g., transforming growth factor-β (TGF-β), interferon, prostaglandin E, tumor necrosis factor, macrophage inflammatory protein-1 (MIP-1), lactoferrin, acidic isoferritins, AcSKDP, and pEEDCK (a synthetic HP5B monomer); or enhancing, e.g., TGF-β, IL-6, IL-4, IL-9, IL-11, MIP-1, MIP-2, leukemia inhibitory factor and *Steel* factor. Pelus *et al. Experimental Hematology* **1994, 22:**239-247. Stimulatory biomolecules have been found to promote division of particular cell lineages. For example, G-CSF derives neutrophil production, while erythropoietin promotes formation of erythrocytes.

A number of these biomolecules and additional agents have been found to induce the mobilization of hematopoietic stem cells.

A single injection of IL-8 has been shown to induce mobilization of pluripotent stem cells that are able to provide permanent reconstitution of myeloid cells and of T and B lymphocytes. Laterveer *et al. Blood* **1995, 85**(8):2269-2275. IL-8 belongs to a family of pro-inflammatory molecules called chemokines. This family has been divided into two subfamilies, the CXC and CC chemokines, based on whether the first two cysteine residues in a conserved motif are adjacent to each other or are separated by an intervening residue. In general, CXC, which include IL-8, melanoma growth-stimulating activity (MGSA) and platelet factor 4 (PF4), are potent chemoattractants and activators of neutrophils but not monocytes. In contrast, CC chemokines, which include RANTES, monocyte chemotactic protein 1 (MCP-1) and MIP-1, are chemoattractants for monocytes but not neutrophils.

Stem cell inhibitors (SCIs) such as the CC chemokines, murine and human MIP-1α (LD78), have also been shown to enhance the release and mobilization of cells into the peripheral blood. WO 94/28916; Simm *et al. Blood* **1994, 84**:2937.

Increased mobilization of stem cells in patients treated with sequentially administered interleukin-3 and GM-CSF compared with GM-CSF alone has been reported by Brugger et al. *Blood* **1992, 79**:1193-1200. In addition, it has been shown that the absolute number of peripheral blood progenitor cells can be expanded *in vitro* by culture in a cocktail of cytokines, usually including SCF, IL-3, and either IL-6 or IL-1. Bodine, D. *Experimental Hematology* **1995, 23**:293-295.

SK&F 107647, a hematoregulatory agent containing an ethylene bridge in place of the cysteine bridge of HP5B, has been demonstrated to be a potent stimulator of *in vitro* myelopoiesis. Pelus *et al. Experimental Hematology* **1994, 22**:239-247. Injection of SK&F 107647 in normal mice resulted in a two- to six-fold increase in serum colony-stimulating activity. Administration of this agent over 4 days resulted in significant increases in the number of granulocyte-macrophage, erythroid, and multipotential progenitor cells, as well as stimulating their cell cycle rates.

It has also been found that pretreatment with stem cell stimulating factor such as G-CSF can expand the pool of progenitor cells susceptible for mobilization by these agents, further increasing their mobilizing effect. For example, the combination of MIP-1α with G-CSF was found to increase white cell count in the blood as compared to G-CSF alone. Simm *et al. Blood* **1994, 84**:2937. Co-administration of SCI with G-CSF caused the enhanced mobilization of a number of cell types including neutrophils, monocytes, eosinphils, lymphocytes and basophils. WO 94/28916. Administration of G-CSF alone had no effect on the release of eosinphils or basophils after 2 days of administration. Similar effects were observed when other agents such as GM-CSF, f-MET-Leu-Phe or IL-8 were coadministered with SCIs.

New chemokines have now been identified which also mobilize stem cells in an animal. These chemokines can be administered alone, or in combination with a colony stimulating factor or hemoregulatory agent to enhance mobilization of stem cells.

An object of the present invention is to provide novel chemokines for the mobilization of stem cells in an animal.

Another object of the invention is to provide a method of mobilizing stem cells.

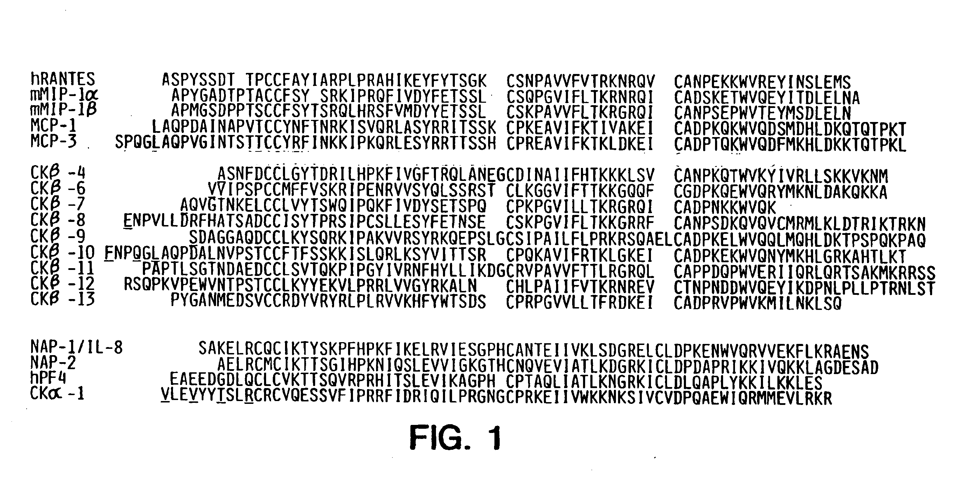

Figure 1 shows the sequence and alignment of the novel chemokines with known chemokines.

In recent years, the availability of recombinant cytokines and the use of hematopoietic stem cell support have resulted in the widespread application of high-dose chemotherapy regimens designed to improve the success of cancer therapy. Despite significant advances, however, delayed recovery of hematopoiesis remains an important source of morbidity and mortality for patients treated with this approach. Since their discovery over 20 years ago, peripheral blood hematopoietic progenitor cells (PBPCs) have been increasingly used to supplement and even replace bone marrow as the source of hematopoietic support in a variety of situations.

Purified populations of cells are increasingly being used therapeutically and it would therefore be advantageous to be able to increase the number of circulating blood cells. It is useful to be able to harvest hematopoietic cells prior to chemotherapy or radiotherapy, thus, protecting them from harmful effects of this therapy; after therapy, the cells can be returned to the patient. It would therefore be highly beneficial to provide an agent which promoted the release and mobilization of a number of hematopoietic cells. Such an agent would be useful for enhancing the response to infection.

Peripheral blood cell transplantation is an important procedure in the treatment of cancer patients with high dose chemotherapy. In such treatment, patients are treated to induce clinical remission of their cancer, then during the remission, successive treatment with CSF, for example, by priming with cyclophosphamide then administration of G-CSF, causes eventual mobilization of cells from the bone marrow to the peripheral circulation for harvesting of leukophoresed blood; then the patient is given high dose chemotherapy or radiotherapy and the resultant bone marrow failure is compensated for by infusion of the stored blood or cells collected previously. This procedure may be modified by the omission of the initial induction of remission, and whole blood may be collected rather than leukophoresed blood. The mobilization effects of the present invention makes it a candidate both to replace CSFs in such cancer treatment regimes, and also to complement the mobilization effects of CSFs in combined treatments.

The two subfamilies of chemokines (CXC and CC) are ever expanding and presumably the individual members have similar, if slightly divergent, functions. The chemokines disclosed in the present invention are new members of the CC subfamily and are structurally similar to MCP-1, MCP-3, hRANTES, mMIP-1α, and mMIP-1β (Figure 1). The effect of these chemokines in inducing leukophilia will find clinical and veterinary application in all utilities where the raising of hematopoietic cell levels is important. For example, a chemokine of the present invention can be used to enhance immune responses against chronic infections, particularly parasitic and bacterial infections. It may also have a role in promoting wound healing.

The chemoattractant activity of these chemokines can be boosted by pretreatment with a colony stimulating factor such as G-CSF or GM-CSF. Alternatively, the hematoregulatory peptides SK&F 107647 (currently in clinical trials), FLT-3 ligand (Immunex) or any other G-CSF mimetics (peptide and non-peptide) may be used. These stimulants may have an even more dramatic effect on these novel chemokines than on those already known due to their slight structural differences. For example, CKB-6 in combination with G-CSF was effective as a mobilizing factor. As known in the art, these peptides are useful in stimulating myelopoiesis in patients suffering from reduced myelopoietic activity, including bone marrow damage, agranulocytosis and aplastic anemia. Also included are patients who have depressed bone marrow function due to immunosuppressive treatment to suppress tissue reactions (i.e., bone marrow transplant surgery). They may also be used to promote more rapid regeneration of bone marrow after cytostatic chemotherapy and radiation therapy for neoplastic and viral diseases. There may also be a value where patients have serious infections due to a lack of immune response following bone marrow failure.

The hematopoietic stem cells released and harvested in the manner described above may be useful for subsequent *in vitro* and *ex vivo* manipulations to deliver gene products in gene therapy. Another embodiment is co-administration with cytotoxic drugs.

The following examples are provided for illustrative purposes only and are not intended to limit the invention.

### EXAMPLES

### Example 1: Mobilization Assay for Novel Chemokines as Single Agents

A panel of novel chemokines will be tested as individual stem cell mobilization agents in BDF 1 mice. These chemokines include, but should not be limited to: Ckβ-4, Ckβ-6, Ckβ-7, Ckβ-8, Ckβ-9, Ckβ-10, Ckβ-11, Ckβ-12, Ckβ-13, and Ckα-1. Each agent will be assayed in concentrations of 50, 10, and 2 µg/mouse and administered via SC, IM, or a PO route. The kinetics of chemokine mobilization of stem cells will be monitored in 15 minute intervals over a period of 60 minutes by collecting blood samples from the mice by cardiac puncture. The mobilized stem cells will be collected by a densing gradient (Lympholyte M). Cells are washed then frozen for future usage. The mobilization profile of the blood differentials will be assessed using a Technicon H1 hematology analyzer. Mobilization of inflammatory cells such as PMN's, eosinophils, and basophils will be taken into account when evaluating the overall potential inflammatory profile. The chemokine IL-8, which mobilizes hematopoietic stem cells as a single factor, will be included in these studies as a positive control.

### Example 2: Mobilization Assay for Novel Chemokines in Combination with Hematostimulants

In these studies, hematostimulants will be assayed in combination with the aforementioned chemokines as mobilization factors. These agents include: G-CSF, GM-CSF, SK&F 107647, and FLT-3 ligand. However, any G-CSF mimetic (hematostimulants which are not colony stimulating factors like G-CSF or GMCSF, but have hematopoietic activity) may be used. In combination studies, G-CSF will be administered IP to mice four days prior to the novel chemokines. As in Example 1, the dose of chemokine and time of blood collection will be varied. Combination studies with hematostimulant pre-treatment will utilize MIP-1α as the positive control.

### Example 3: CFU Assay

Blood samples collected during the mobilization phase will be assessed for colony forming units (CFU-GM) at days 7 and 14. Cells are adjusted to 2X10⁶ cells/ml in McCoys medium with 15 x FBS serum. A single layer agar system utilizing the following is used: McCoys medium enriched with nutrients (NaHCO₃, pyruvate, amino acids and vitamins); 0.3% Bacto agar. To this is added cells from the blood samples (final concentration = 2X10⁵ cells/ml). The agar plates are incubated at 37°C, 5% CO₂ for 7 days. Colonies of proliferating cells (CFU-GM) are counted utilizing a microscope. In addition, early hematopoietic high proliferative potential (HPP) progenitors, will be counted in the day 14 CFU cultures.

## Claims

1. Use of an effective amount of a polypeptide comprising SEQ ID NO: 7 in the manufacture of a medicament for administration to an animal in need thereof for mobilising hematopoietic stem cells.

2. The use of claim 1, wherein said medicament is adapted for administration with a cytotoxic drug.

3. The use of claim 1, wherein said medicament is adapted for administration with a colony stimulating factor.

4. The use of claim 1, wherein said medicament is adapted for administration with a hemoregulatory agent.

5. The use of any proceeding claim, wherein said polypeptide consists of the polypeptide of SEQ ID NO: 7

6. A chemokine comprising SEQ ID NO: 7 capable of mobilizing stem cells.

7. A chemokine comprising SEQ ID NO: 1 capable of mobilizing stem cells.

8. A chemokine comprising SEQ ID NO: 2 capable of mobilizing stem cells.

9. A chemokine comprising SEQ ID NO: 3 capable of mobilizing stem cells.

10. A chemokine comprising SEQ ID NO: 4 capable of mobilizing stem cells.

11. A chemokine comprising SEQ ID NO: 5 capable of mobilizing stem cells.

12. A chemokine comprising SEQ ID NO: 6 capable of mobilizing stem cells.

13. A chemokine comprising SEQ ID NO: 8 capable of mobilizing stem cells.

14. A chemokine comprising SEQ ID NO: 9 capable of mobilizing stem cells.

15. A method of mobilizing stem cells in an animal comprising administering to an animal an effective amount of a chemokine comprising SEQ ID NO: 7.

16. A method of mobilizing stem cells in an animal comprising administering to an animal an effective amount of a chemokine comprising SEQ ID NO: 1.

17. A method of mobilizing stem cells in an animal comprising administering to an animal an effective amount of a chemokine comprising SEQ ID NO: 2.

18. A method of mobilizing stem cells in an animal comprising administering to an animal an effective amount of a chemokine comprising SEQ ID NO: 3.

19. A method of mobilizing stem cells in an animal comprising administering to an animal an effective amount of a chemokine comprising SEQ ID NO: 4.

20. A method of mobilizing stem cells in an animal comprising administering to an animal an effective amount of a chemokine comprising SEQ ID NO: 5.

21. A method of mobilizing stem cells in an animal comprising administering to an animal an effective amount of a chemokine comprising SEQ ID NO: 6.

22. A method of mobilizing stem cells in an animal comprising administering to an animal an effective amount of a chemokine comprising SEQ ID NO: 8.

23. A method of mobilizing stem cells in an animal comprising administering to an animal an effective amount of a chemokine comprising SEQ ID NO: 9.

24. The method of claims 15-23 further comprising administering a colony stimulating factor.

25. The method of claims 15-23 further comprising administering a hematoregulatory agent.
